# EUROPÄISCHE PATENTSCHRIFT

(11) **EP 0 431 124 B1**
(45) Veröffentlichungstag und Bekanntmachung des Hinweises auf die Patenterteilung: **22.05.1996**
(21) Anmeldenummer: 90909720.6
(22) Anmeldetag: 22.06.1990
(51) Int. Cl.: C08G 18/36, C08G 18/38, C08G 75/00, C08G 63/48, C08G 63/68, C08G 64/00, C08G 59/40

(54) **NEUARTIGE KUNSTSTOFFE AUF FETTSÄUREBASIS**
NEW PLASTICS BASED ON FATTY ACIDS
MATIERES PLASTIQUES NOUVELLES A BASE D'ACIDES GRAS

(30) Priorität: 29.06.1989 DE 3921306
(43) Veröffentlichungstag der Anmeldung: 12.06.1991
(73) Patentinhaber: Dr. Frische GmbH, D-63755 Alzenau (DE)
(72) Erfinder: FRISCHE, Rainer, D-6000 Frankfurt am Main (DE); VOLKHEIMER, Jürgen, D-6200 Wiesbaden (DE); WOLLMANN, Klaus, D-651 Eschhofen (DE); SCHOMANN, Herrmann, D-6070 Langen (DE); SCHNEIDER, Judith, D-6000 Frankfurt am Main (DE); ACH, Alexander, D-6000 Frankfurt am Main (DE); GROSS-LANNERT, Renate, D-6057 Dietzenbach (DE); BEST, Bernd, D-6082 Mörfelden (DE)
(74) Vertreter: Reichel, Wolfgang, Dipl.-Ing.
(86) Internationale Anmeldenummer: EP9000995
(87) Internationale Veröffentlichungsnummer: WO9100305

(56) Entgegenhaltungen:
- DE-A- 1 495 251
- DE-B- 1 049 575
- GB-A- 23 668
- GB-A- 1 153 557
- US-A- 3 001 958
- US-A- 3 388 100
- US-A- 4 535 142

## Beschreibung

Die Erfindung bezieht sich auf thermoplastische lineare Polymere auf der Basis von aus natürlichen Fetten und Öleen gewinnbaren Fettsäuren.

Natürliche Öle und Fette dienen nicht nur als Ausgangsmaterial zur Erzeugung einer Vielzahl technisch wichtiger Grundstoffe, sie können unter bestimmten Voraussetzungen in erheblich verstärktem Maße auch zur Herstellung von Polymeren verwendet werden. Derzeit beruht die Herstellung von Polymeren aus Fetten und Ölen im wesentlichen auf drei verschiedenen Möglichkeiten.

Die erste Möglichkeit macht sich die Beobachtung zunutze, daß mehrfach ungesättigte Fette und Fettsäuren beim Erhitzen oder bei Zutritt von Luftsauerstoff zur Polymerisation neigen. Diese Eigenschaften spielen beispielsweise bei der Herstellung von Linoleum und bei der Härtung von Lacken und Farben (Ölfarben) sowie bei Dichtmassen, z.B. Fensterkitt, eine Rolle. Der Anwendungsbereich beschränkt sich hierbei naturgemäß auf Öle mit einem hohen Gehalt an mehrfach ungesättigten Fettsäuren, sogenannte trocknende Öle wie Leinöl, Holzöl oder Nußöl.

Eine zweite Möglichkeit besteht darin, Öle und Fette zu verwenden, die aufgrund ihrer Zusammensetzung in überwiegendem Maße zwei oder mehr Alkoholgruppen pro Triglyceridmolekül enthalten, und diese mit entsprechenden reaktiven Verbindungen, z.B. mit Diisocyanaten, zu polymeren Strukturen zu verarbeiten. Für derartige Umsetzungen sind z.B. Ricinusöl oder hydriertes Ricinusöl, die direkt als Triglyceride eingesetzt werden können, geeignet. Auch hier ist jedoch die Anwendungsbreite der Methode durch die zur Verfügung stehenden Öle und Fette begrenzt und außerdem läßt die direkte Verwendung von Triglyceriden meist nur die Bildung vernetzter und damit thermoplastisch nicht verarbeitbarer Polymere zu.

So ist es aus GB-A-1153557 bekannt, faktisähnliche Polymerisate durch Umsetzung von Glycolestern von ungesättigten natürlich vorkommenden oder synthetischen Fettsäuren und Hydroxyfettsäuren mit sowohl Schwefeldichlorid als auch Phosphorhalogeniden herzustellen.

Aus US-A-4 535 142 ist die Herstellung von vernetzten Überzügen durch Versprühen und Aushärten von Isocyanat/ Glycol-diricinoleat-Gemischen bekannt. Die Aushärtung erfolgt unter Vernetzung, da in sämtlichen beschriebenen Fällen (Beispiele 1 bis 27) als Isocyanat-Komponente ein Triisocyanat ("Mondur® CB-75") verwendet wird.

Aus DE-B-1 049 575 ist die Herstellung eines thixotropen Urethanöls direkt aus nativen Ölen (Triglyceriden), deren Fettsäuremuster einen hohen Anteil an ungesättigten Fettsäuren aufweist, bekannt. Diese Öle werden mit Glycol und einem Katalysator (Lithiumnaphthenat) zu Gemischen aus Glycerin- und Glycolestern und letztere mit einem Diisocyanat umgesetzt.,

Aus DE-A-1 495 251 ist ein Verfahren zur Herstellung von Polyesterharzen bekannt. Dabei wird ein hochmolekularer Terephthalsäurediolester in einem ersten Schritt durch Umestern mit Propandiol zu niedermolekularen Verbindungen abgebaut Im zweiten Schritt wird dieses Oligomerengemisch dann mit Phthalsäureanhydrid und Ricinolsäure wieder zu höhermolekularen Verbindungen kondensiert. In diesen Polyesterharzen sind Säurekomponente und Alkoholkomponente über Esterbindungen miteinander in statistischer Reihenfolge verknüpft. Das Kondensationsprodukt ist ein Harz und kein thermoplastischer Polyester.

Schließlich ist aus US-A-3 001 958 eine Teil-Umesterung von Ricinusöl mit Glycol und die anschließende Umsetzung des Produktes der Umesterung mit einem überschuß an einem Diisocyanat zu einem Vorpolymerisat, das anschließend mit einem N-substituierten Diethanolamin zu einem trocknenden Öl umgesetzt wird, bekannt.

Die dritte Möglichkeit besteht darin, die in Ölen und Fetten enthaltenen Fettsäuren durch Spaltreaktionen, wie z.B. Ozonolyse, zu diaktiven Fettsäurespaltprodukten, wie z.B. Dicarbonsäuren, aufzuspalten, wobei sich die reaktiven Gruppen, z.B. Carbonsäuregruppen jeweils am Anfang und am Ende einer Kohlenwasserstoffkette befinden. Nach dieser Methode kann in technischem Maßstab aus Ölsäure Azelainsäure hergestellt werden. Die Dicarbonsäuren können anschließend mit Diaminen zu Polyamiden oder mit Diolen zu Polyestern umgesetzt werden. Auch die Spaltung der Ricinolsäure (aus dem Ricinusöl) und Umsetzung eines der Spaltprodukte zu 11-Aminoundecansäure sowie deren Polykondensation zu Nylon R 11 (Rilsan) wird von der chemischen Industrie durchgeführt. Obwohl auf dem Weg über diese diaktiven Fettsäurespaltprodukte und über andere diaktive Fettsäurespaltprodukte auch thermoplastische Polymere erzeugt werden können, weist auch dieses Verfahren entscheidende Nachteile auf. Zum einen ist die Spaltung der Fettsäuren zu diaktiven Spaltprodukten relativ aufwendig und verlustreich, zum anderen entstehen hierbei erhebliche Mengen verschiedenster Nebenprodukte, vor allem aliphatische Monocarbonsäuren mit technisch weniger interessanter Kettenlänge, wobei sich auch die anschließenden Reinigungsoperationen entsprechend schwierig gestalten.

Aus US-A-3 388 100 ist ein Polymerisat bekannt, das durch Umesterung eines Diisocyanats mit einer praktisch linearen organischen Verbindung, die mit NCO-Gruppen umsetzbare Wasserstoffatome aufweist, sowie Kettenverlängerern hergestellt worden ist.

Die Kettenverlängerer können Diolamide, hergestellt aus einem organischen Diamin und einer Hydroxycarbonsäure, sein. Sie werden anstelle von zuvor verwendeten Kettenverlängerern, wie einfachen Glycolen, wie beispielsweise Butandiol, eingesetzt. Wie sämtliche Beispiele dieser Patentschrift zeigen, ist kein einziges Polymerisat aus natürlichen Ölen oder Fetten bzw. den daraus erhältlichen Fettsäuren hergestellt. Vielmehr stellen die Ausgangsmaterialien ausschließlich Erdölprodukte dar. Native Ausgangsmaterialien werden in der Patentschrift überhaupt nicht erwähnt. Abgesehen davon, werden Aufbau und Eigenschaften der erhaltenen Kunststoffe wesentlich durch die lineare organische Verbindung, deren bereits vorhandene Kette verlängert werden soll, die also selbst ein Polymerisat, wie beispielsweise ein zwei Hydroxylendgruppen aufweisender Polyester ist, mitbestimmt.

Die angeführten Beispiele zeigen bereits das grundlegende Interesse an der Verwendung natürlicher Fette und Öle in der Kunststoffindustrie.

Aufgabe der vorliegenden Erfindung war es daher, die insbesondere in natürlichen Fetten und Ölen vorhandenen Fettsäuren mit funktionellen Gruppen wie Doppelbindungen oder Hydroxylfunktionen, die sich für die Umsetzung zu Polymeren eignen, auf neue Weise zugänglich zu machen und somit das Anwendungsspektrum für Fette und Öle in der kunststofferzeugenden Industrie zu erweitern. Grundlage der vorliegenden Erfindung war hierbei die Überlegung, daß sich Moleküle mit mindestens zwei reaktionsfähigen Gruppen, z.B. Doppelbindungen, Epoxidgruppen oder Hydroxylgruppen, mit anderen geeigneten bifunktionellen Verbindungen, z.B. Diisocyanaten, zu linearen Polymeren umsetzen lassen. Dabei muß sichergestellt werden, daß pro Monomer jeweils zwei reaktive Gruppen abreagieren; reagieren weniger Gruppen pro Molekül, so erfolgt Kettenabbruch, reagieren mehr als zwei, so erfolgt Vernetzung.

Gegenstand der vorliegenden Erfindung sind demnach thermoplastische lineare Polymere, die dadurch erhältlich sind, daß man zuerst aus ungesättigten und/oder hydroxylgruppenhaltigen, aus natürlichen Fetten und Ölen gewinnbaren Fettsäuren oder deren Estern oder Gemischen verschiedener solcher Fettsäuren und Ester durch Reaktion in stöchiometrischem Mischungsverhältnis mit bifunktionellen Verbindungen in Form von Diolen, Diaminen oder Aminoalkoholen, wobei OH-Gruppen in den Diolen durch SH-Gruppen ersetzt sein können, und gegebenenfalls durch anschließende Umsetzung vorhandener ethylenischer Doppelbindungen zu Amino-, Hydroxyl- oder Epoxidgruppen, Difettsäurediamide, Difettsäurediester, Difettsäureamidester, Monofettsäureamidamine oder Monofettsäureamidalkohole bzw. die entsprechenden Thioverbindungen als Monomerbausteine herstellt, die wenigstens zwei funktionelle Gruppen in Form von ethylenischen Doppelbindungen, Hydroxylgruppen, Epoxidgruppen oder Aminogruppen enthalten, und danach diese Monomerbausteine in äquimolaren Mengen mit bifunktionellen Verbindungen in Form von Dischwefeldichlorid, Diisocyanaten, aktivierten Dicarbonsäuren oder Dicarbonsäurederivaten, entsprechenden Thiosäuren, Phosgen oder Diketenen oder, wenn die Monomerbausteine Epoxidgruppen aufweisen, in Form von Diolen oder den entsprechenden Thioverbindungen, Dicarbonsäuren, Aminoalkoholen oder Diaminen weiter umsetzt.

Zur Herstellung der erfindungsgemäßen Polymere geht man von ungesättigten und/oder amino- und/oder hydroxylgruppenhaltigen, aus natürlichen Fetten und Ölen gewinnbaren Fettsäuren, zweckmäßig mit Kettenlängen von 10 bis 24 Kohlenstoffatomen, oder deren Derivaten, vorzugsweise Estern, aus. In der Regel wird man als Ausgangsmaterial natürliche Fette und Öle verwenden, die bereits einen besonders hohen Gehalt an einer derartigen Fettsäure, z.B. an Ölsäure oder Ricinolsäure besitzen. Sofern solche Fette und Ole Fettsäuren mit einer oder mehreren Doppelbindungen besitzen, können Hydroxyl- oder auch Aminogruppen nachträglich, unter Umständen sogar erst nach der Umsetzung des Ausgangsmaterials mit den bifunktionellen ester- oder amidbildenden Substanzen, im Falle der Hydroxylgruppen beispielsweise durch Oxidation des Ausgangsmaterials mit Peressigsäure oder Perameisensäure, erzeugt werden, wodurch das Spektrum der nachfolgend umsetzbaren Verbindungen mit Fettsäureestern wesentlich erweitert werden kann. Öle, die sich für das erfindungsgemäße Verfahren besonders gut eignen, sind beispielsweise das Öl aus den Samen der Euphorbia lathyris, Olivenöl, Ricinusöl und hydriertes Ricinusöl, ölsäurereiches Sonnenblumenöl, insbesondere der Art "High oleic", erucasäurereiches Rapsöl, das Öl der Purgiernuß oder Seetieröle, wie Fisch- oder Waltranöl.

Das Ausgangsmaterial wird direkt mit den Vertretern der ersten Gruppe bifunktioneller Substanzen umgesetzt. Geeignete bifunktionelle Verbindungen können sein: Diole, Diamine oder Aminoalkohole. Statt der Diole können auch entsprechende Thioverbindungen eingesetzt werden, in denen eine oder beide OH-Gruppen durch SH-Gruppen ersetzt sind. Zur Umsetzung mit Diolen wird das Ausgangsmaterial zweckmäßig in vorgereinigter Form eingesetzt. Wie aus der parallel eingereichten Patentanmeldung DE 40 19 089 A1 hervorgeht, können für die Umsetzung mit Diaminen oder Aminoalkoholen auch rohe ungereinigte Öle und Fette problemlos verwendet werden.

Diole, die sich in dem erfindungsgemäßen Verfahren verwenden lassen, sind z.B. primäre und sekundäre aliphatische, cycloaliphatische, aliphatisch-aromatische und aromatische Diole, vorzugsweise mit 2 - 44 Kohlenstoffatomen. Bevorzugt eingesetzt werden 2-Butin-1,4-diol, 2-Buten-1,4-diol, 1,5-Pentandiol, 1,6-Hexandiol, Neopentylglycol, N,N-Diethylamino-propandiol-2,3 oder Hydroxypivalinsäureneopentylglycolester. Besonders bevorzugt sind 1,4-Butandiol, 1,2-Propandiol, 1,10-Decandiol und Glycol. Als Thioverbindung kommt beispielsweise 2-Mercaptoethanol in Frage.

Als Diamine oder Aminoalkohole eignen sich primäre und sekundäre aliphatische, cycloaliphatische, aliphatisch-aromatische und aromatische Diamine bzw. Aminoalkohole, vorzugsweise mit 2 - 44 Kohlenstoffatomen. Hierzu gehören u.a. beispielsweise auch dimere Fettsäuren ausnatürlichen Fetten und Ölen. Zwischen den beiden Aminofunktionen der Diamine können sich in der Kohlenwasserstoffkette oder am cycloaliphatischen oder aromatischen Rest zusätzliche strukturelle Elemente bzw. weitere funktionelle Gruppen, z.B. Ethergruppen, Diamidgruppierungen, Aminogruppen, Ketogruppen oder Sulfongruppen, befinden. Bevorzugt eingesetzte Diamine sind 1,2-Diaminoethan, 1,3-Diaminopropan, 1,6-Diaminohexan, 1,8-Diaminooctan, Piperazin, Diethylentriamin, 4,7,10-Trioxatridecan-1,13-diamin, 3,3'-Diaminodiphenylsulfon, 3,3'-Dimethyl-4,4'-diaminodicyclohexylmethan und handelsübliche Etherdiamine der Formel 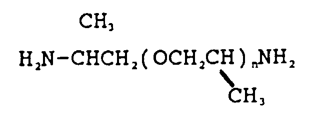 wobei n eine ganze Zahl von 1 bis 2000 sein kann. Besonders bevorzugt werden 1,2-Diaminoethan und 1,6-Diaminohexan eingesetzt. Bevorzugte Aminoalkohole sind 2-Aminoethanol und 3-Aminopropanol.

Diole, Diamine und Aminoalkohole werden, wenn als Reaktionsprodukte Difettsäurediester, Difettsäurediamide bzw. Difettsäureamidester erhalten werden sollen, in stöchiometrischem Mischungs-verhältnis umgesetzt, so daß pro Fettsäurecarboxylgruppe eine Amino- bzw. Alkoholgruppe reagieren kann. Im Falle der Diamine ist das Mischungsverhältnis jedoch nicht so kritisch, da sich überraschenderweise erst bei einem sehr großen Überschuß an Aminofunktionen bevorzugt das Monoamid bildet.

Im Falle der Umsetzung mit Aminoalkoholen bzw. Diaminen sind auch die Monofettsäureamide oben genannter Fettsäuren wünschenswerte Reaktionsprodukte, da in derartigen Verbindungen die OH-Gruppe des Aminoalkohols bzw. die freie Aminogruppe des umgesetzten Diamins neben der funktionellen Gruppe der Fettsäure als weitere Kopplungsgruppe dienen kann, über die eine Verknüpfung der Monomeren zu Polymeren möglich ist. Durch Verwendung eines Überschusses des Aminoalkohols erhält man wegen der höheren Reaktivität der Aminofunktion nahezu ausschließlich Monofettsäureamide. Die Bildung von Monofettsäureamidaminen erfolgt, wie oben ausgeführt, ebenfalls mit einem hohen Überschuß des Diamins.

Gegebenenfalls kann die Umsetzung auch in einem geeigneten Lösungsmittel erfolgen, um eine homogene Reaktionsführung zu gewährleisten. Geeignet sind je nach Reaktionstyp sowohl polare wie unpolare Lösungsmittel, insbesondere Methanol, Ethanol, Propanol, Butanol, Toluol, Xylol oder Petrolether.

Die Umsetzung kann in einem Temperaturbereich zwischen 20 und 300°C erfolgen, bevorzugt wird jedoch ein Bereich zwischen 50 und 200°C, da die Reaktionszeiten in diesem Temperaturbereich für eine praktische Durchführung des Verfahrens noch nicht zu lang sind.

Die Reaktion wird vorsorglich in einem geschlossenen System, z.B. einem Autoklaven, durchgeführt. Die Reaktion kann ohne besonderen Aufwand erfolgen, wird jedoch bevorzugt unter Inertgasatmophäre, beispielsweise Argon oder Stickstoff, gegebenenfalls auch der Lösungsmittelatmosphäre, durchgeführt, da hierdurch größere Sicherheit gegenüber unerwünschten Nebenreaktionen wie der Oxidation der Ausgangsmaterialien gewährleistet wird.

Falls erforderlich können dem Reaktionsgemisch auch Katalysatoren, z.B. Ammoniumchlorid oder p-Toluolsulfonsäure, zugefügt werden. Ebenso können weitere übliche Hilfs- und Zusatzstoffe wie Polymerisationshemmer und Antioxidantien, z.B. Ascorbinsäure oder Glukose zugesetzt werden.

Nach Beendigung der Reaktion können die Reaktionsprodukte, gegebenenfalls nach Abziehen des Lösungsmittels, durch einfache oder fraktionierte Kristallisation abgetrennt und falls erforderlich anschließend aus geeigneten Lösungsmitteln umkristallisiert werden. Als Lösungsmittel für die Kristallisation eignen sich sowohl polare wie unpolare Verbindungen. Bevorzugt wird aus Methanol oder Ethanol umkristallisiert. Unter Umständen genügt auch bereits ein einfacher Waschvorgang, um reine Reaktionsprodukte zu erhalten. In bestimmten Fällen kann die Reaktionsmischung vorteilhaft einer Lösungsmittelheißdampf-Extraktion unterworfen werden, um die Reaktionsprodukte in reiner Form zu erhalten.

Sofern die hydroxyl- oder aminogruppenhaltigen Fettsäuren nicht bereits in den natürlichen Fetten und Ölen vorlagen bzw. durch Oxidation oder andersartige Umsetzung des Ausgangsmaterials erzeugt wurden, können die nach dem beschriebenen Verfahren erhaltenen Zwischenprodukte, sofern sie Doppelbindungen enthalten, auch noch vor oder nach einem möglichen Reinigungsschritt zu entsprechenden Verbindungen funktionalisiert werden. In diesem Fall erhält auch die Bildung von Epoxidgruppen als reaktive Gruppe für weitere Umsetzungen Bedeutung.

Monomere, die nach diesem Verfahren erhältlich sind und die für eine weitere Umsetzung zu Kunststoffen besonders interessant sind, sind vor allem die Diester, Diamide und Amidester derjenigen Derivate von Fettsäuren, insbesondere der Stearinsäure, die eine oder mehrere Doppelbindungen, Epoxid-, Amino- oder Hydroxylgruppen möglichst nahe am Kettenende, in der Regel im Bereich der Kohlenstoffatome 9 - 16 besitzen. Solche Derivate sind beispielsweise Ölsäure, Ricinolsäure, 9-, 10- oder 12-Hydroxystearinsäure, 9,10-Epoxystearinsäure und Linolsäure, Linolensäure und Erucasäure sowie die aus diesen ableitbaren Mono- und Oligohydroxy- oder Mono- und Oligoepoxyverbindungen, insbesondere die 9,10-Dihydroxystearin- säure. Die Diester, Diamide und Amidester müssen dabei nicht symmetrisch aufgebaut sein, sondern können auch verschiedenartige Fettsäurereste enthalten. Interessant sind ferner auch die Monofettsäureamide der genannten substituierten Säuren, die bei der Umsetzung mit Aminoalkoholen bzw. Diaminen erhalten werden können.

Die nach diesem Verfahren erhältlichen Monomere besitzen jeweils mindestens zwei funktionelle Gruppen, die, wie in den Diamiden, Diestern oder Amidestern, entweder Teil des Fettsäurerestes sind, oder die, wie es bei den durch Umsetzung mit den Aminoalkoholen bzw. Diaminen erhaltenen Monofettsäureamiden der Fall ist, teils von der Fettsäure und teils von dem Aminoalkohol bzw. Diamin zur Verfügung gestellt werden. Solche Monomerverbindungen lassen sich mit geeigneten bifunktionellen Verbindungen unter bekanntermaßen geeigneten Bedingungen zu linearen Polymeren verknüpfen.

Erfindungsgemäß lassen sich beispielsweise die Diamide, Diester und Amidester von Fettsäuren, die mehrere Doppelbindungen in den Fettsäureresten enthalten, z.B. Verbindungen der Ölsäure wie das Diölsäureethylendiamid, mit Dischwefeldichlorid zu Polymeren umsetzen.

Monomere, die wenigsten zwei Hydroxyfunktionen bzw. eine Aminogruppe und eine Hydroxyfunktion besitzen, können mit Diisocyanaten, wie sie in der Kunststoffherstellung bekannt sind, z.B. mit Hexamethylendiisocyanat, Methylendiphenylendiisocanat (MDI) oder den unter den Handelsnamen Desmodur^{(R)} E14 oder T80 erhältlichen Diisocyanaten umgesetzt werden. Andere zur Umsetzung dieser Monomerklasse geeignete bifunktionelle Verbindungen sind aktivierte Dicarbonsäuren bzw. Dicarbonsäurederivate, z.B. Säurechloride, Ester, Anhydride, Azide oder Nitrile, entsprechende Thiosäuren oder die Diketene. Bevorzugt eingesetzte Vertreter solcher bifunktionellen Verbindungen sind Phthalsäuredichlorid, Adipinsäuredichlorid, Maleinsäuredichlorid oder Phosgen.

Zu einer weiteren sehr interessanten Klasse von Polymeren gelangt man, wenn man die Epoxyverbindungen der Diamide, Diester oder Amidester mit Diolen, z.B. 1,4-Butandiol oder den entsprechenden Thioverbindungen umsetzt. In diesem Falle werden die entsprechenden Monomere über Ether- bzw. Thioethergruppen miteinander verknüpft, die sich in Nachbarschaft zu einer Hydroxylgruppe befinden. Die Epoxyverbindungen lassen sich außerdem über Dicarbonsäuren oder über Aminoalkohole oder Diamine verknüpfen.

In den erfindungsgemäßen Ausführungsformen der Diester, Diamide und der Amidester liegen die Fettsäuren in den Monomeren über die Carboxylgruppe verknüpft in einer Kopf-Kopf-Anordnung vor. Dies stellt insbesondere im Falle der hydroxylgruppenhaltigen Diamide in Verbindung mit Diisocyanaten als monomerverknüpfenden Reaktanten ein ganz neuartiges Bauprinzip von Polymeren dar und führt zu der vollkommen neuen Kunststoffklasse der Polyamidurethane. Die Art der Verknüpfungen führt hierbei zu einer besonders starken polaren Wechselwirkung der Polymerketten, ohne dabei die thermoplastischen Eigenschaften des Kunststoffs zu beeinträchtigen. Ein Beispiel hierfür ist die Umsetzung von Bis-12-Hydroxystearinsäure-1,2-N,N'-ethylendiamid mit Hexamethylendiisocyanat. Ähnlich interessante Kunststoffe mit demselben Bauprinzip, die Polyamidester, erhält man, wenn man die Diisocyanatverbindung durch aktivierte Dicarbonsäuren, z.B. durch Adipinsäuredichlorid ersetzt.

Die Umsetzung der einzelnen Komponenten erfolgt nach den in der Kunststoffherstellung bekannten und dem Fachmann geläufigen Methoden. Bezogen auf die zur Reaktion zu bringenden funktionellen Gruppen werden die einzelnen Komponenten in äquimolaren Mengen eingebracht, und man arbeitet in einem Temperaturbereich zwischen 20 und 180°C, vorzugsweise in der Schmelze und vorsorglich unter einer Inertgasatmosphäre, z.B. von Stickstoff.

Der besondere Vorteil des erfindungsgemäßen Verfahrens liegt in den vielfältigen Möglichkeiten, die dieses System zur Variation der Kunststoffeigenschaften zur Verfügung stellt. Die Eigenschaften lassen sich einerseits über das Bindeglied, das die Fettsäuren über die Carboxylfunktion verknüpft, d.h. also die Diamine, Diole oder Aminoalkohole, zum zweiten durch die Wahl der Fettsäure und schließlich über die bifunktionelle Verknüpfungskomponente, mit der die Monomeren umgesetzt werden, also beispielsweise das Diisocyanat oder das Säuredichlorid, beeinflussen. Weiterhin können auch Mischungen unterschiedlicher Monomere, z.B. Bis-Ricinolsäure-1,2-N,N'-ethylendiamid und Bis-Ricinolsäure-1,6-N,N'-hexamethylendiamid oder Bis-Ricinolsäure-1,2-N,N'-ethylendiamid und Bis-12-Hydroxystearinsäure-1,2-N,N'-ethylendiamid zu Polymeren umgesetzt werden, wodurch zusätzlich Einfluß auf die resultierenden Kunststoffeigenschaften genommen werden kann. Ganz allgemein sind hierbei die gängigen Regeln der klassischen Polymerchemie zu berücksichtigen. So ist festzustellen, daß die Flexibilität der Kunststoffe mit wachsender Kettenlänge des Fettsäurerestes zunimmt, und gleiches gilt auch für die Länge der verschiedenen bifunktionellen Verbindungen, die die verknüpfenden Elemente bilden. Da die in den nativen Fetten und Ölen vorkommenden Fettsäuren, die in der Regel das Hauptausgangsmaterial für die Herstellung der erfindungsgemäßen Kunststoffe darstellen dürften, ihre funktionellen Gruppen im allgemeinen etwa in der Mitte der Fettsäurekette tragen, so z.B. die Ricinolsäure die Hydroxylgruppe an C₁₂ oder die Ölsäure die Doppelbindung zwischen C₉ und C₁₀, enthalten die verknüpften Polymerketten auch immer mehr oder weniger langkettige aliphatische Reste. Derartige aliphatische Restgruppen in Polymeren vermitteln dem Kunststoff einerseits hydrophobe Eigenschaften und haben außerdem die Wirkung von inneren Weichmachern. Ihre Anwesenheit kann daher für die Erzeugung flexibler Kunststoffe von besonderem Vorteil sein. Umgekehrt können jedoch die polaren Bindungstypen, die in diesem Kunststoffsystem auftreten können, dieser Weichmacherwirkung über Wasserstoffbrückenbindungen oder Allophanatbildung entgegenwirken, oder dieser möglicherweise unerwünschte Einfluß der Seitenketten kann über die geeignete Wahl der verknüpfenden bifunktionellen Elemente kompensiert werden. Die enorme Bedeutung, die diesen Verküpfungselementen aufgrund ihrer Variabilität zukommt, zeigt sich beispielsweise im Vergleich mit den Dimeren der Ricinolsäure, deren Einsatz in der Kunststoffindustrie, beispielsweise zur Bildung von Polyestern, wegen der unvermeidlichen Weichmacherwirkung der aliphatischen Seitenketten auf dem Rückgrat der Polymerstruktur nur begrenzt möglich ist.

Die Polarität der Bindungen macht die auf diese Weise erzeugten Kunststoffe wegen der damit einhergehenden günstigen Haftungseigenschaften besonders geeignet als Glasfaserverbundstoff, wobei die niedrige Verarbeitungstemperatur einen weiteren Vorteil dieses Systems darstellt.

Diese beschriebenen Variationsmöglichkeiten erlauben es somit ganz allgemein, Kunststoffe herzustellen, die den jeweiligen Bedürfnissen exakt angepaßt sind. So lassen sich auf diese Weise Kunststoffe erhalten, die sowohl thermoplastisch verarbeitbar, im Spritzgußverfahren verwendbar oder zur Extrusion zu Folien geeignet sind. Auch andere Eigenschaften, wie Reißfestigkeit, Reckfähigkeit, Kerbschlag-Zähigkeit, Glaspunkt oder Kristallinität lassen sich in weiten Grenzen variieren.

Ein weiterer Vorteil der vorliegenden Erfindung ist es, daß es sich bereits bei den über die Carboxylfunktionen verknüpften Fettsäurebausteinen,verglichen mit den sonst üblicherweise bei der Kunststoffherstellung verwendeten Monomeren, um relativ große Moleküle handelt, so daß bei der weiteren Umsetzung mit bifunktionellen reaktiven Verbindungen bereits vergleichsweise wenige Verknüpfungsreaktionen ausreichen, um zu linearen Polymeren ausreichender Länge zu gelangen.

Überraschend wurde außerdem gefunden, daß auch bei einem relativ hohen Gehalt an kettenabbrechenden Verbindungen unter den carboxylverknüpften Fettsäuren, d.h. an Verbindungen, wie sie beispielsweise bei der Reaktion mit einer Fettsäure entstehen, die keine funktionellen Gruppen in der aliphatischen Kette enthält, und in denen somit nur eine funktionelle Gruppe zur Weiterreaktion zur Verfügung steht, dennoch Kunststoffe mit erstaunlich guten Eigenschaften erhalten werden können. Da außerdem Verbindungen ohne funktionelle Gruppen nicht zum Kettenabbruch führen können, sondern sich vielmehr wie ein normales Additiv verhalten, kann bei Einsatz von Ölen und Fetten, in denen der Anteil funktionelle Gruppen tragender Fettsäuren hinreichend hoch ist, unter Umständen sogar auf eine Reinigung und Isolierung der entstandenen Monomeren verzichtet werden.

Darüber hinaus können die carboxylverknüpften Fettsäurebausteine auch in andere Kunststoffsysteme eingebettet werden, in denen sie beispielsweise als Weichmacher fungieren können. So entstehen bei der Reaktion von 1,4-Butandiol als Alkoholkomponente mit Hexamethylendiisocynanat harte und brüchige Kunststoffe. Wird bei dieser Umsetzung ein Teil des Butandiols durch Bis-12-Hydroxystearinsäure-1,2-N,N'-ethylendiamid ersetzt, so erhält man hingegen wesentlich flexiblere und elastischere Kunststoffe.

Wie bereits ausgeführt, ist der Einsatz von Fetten und Ölen als Ausgangsmaterial für die kunststoffherstellende Industrie bisher dadurch beschränkt, daß bei direkter Verwendung von Triglyceriden, die aus Fettsäuren mit geeigneten funktionellen Gruppen aufgebaut sind, lediglich Duroplaste zu erhalten sind oder aber daß eine oxidative Spaltung der Fettsäuren durchgeführt werden muß, bei der ein erheblicher Anteil des zur Verfügung stehenden Fettsäure-materials verloren geht. Durch die Erfindung wird nun zum ersten Mal ein Verfahren zur Verfügung gestellt, mit dessen Hilfe es möglich ist, aus Fetten und Ölen ohne vorhergehende Spaltung der Fettsäurereste eine Vielzahl an Kunststoffprodukten mit unterschiedlichsten Eigenschaften bzw. Eigenschaftskombinationen zu erzeugen. Da es sich bei den vorliegenden Kunststoffen außerdem um Polymere handelt, deren Ausgangsmaterialien zumindest teilweise aus biologischen Quellen gewonnen werden können, ist damit zu rechnen, daß diese Produkte auch leichter abbaubar und somit auf lange Sicht unter dem Gesichtspunkt der Umweltverträglichkeit weitaus positiver zu bewerten sind als die Mehrzahl der bekannten herkömmlichen Kunststoffe.

Die vorliegende Erfindung wird durch die nachfolgenden Beispiele näher erläutert.

### Beispiel 1

### Herstellung von Bis-12-hydroxystearinsäure-1,2-N,N'-ethylendiamid

153 g gehärtetes Ricinusöl und 15 g Ethylendiamin wurden 5 Stunden lang unter Stickstoffatmosphäre bei 140°C im Autoklaven gerührt. Das Reaktionsprodukt wurde aus heißem Methanol umkristallisiert. Schmelzpunkt 142 bis 145°C, Ausbeute: 106,5 g.

### Beispiel 2

### Herstellung von Bis-12-hydroxystearinsäure-1,6-N,N'-hexamethylendiamid

5,1 g gehärtetes Ricinusöl und 0,97 g Hexamethylendiamin wurden 5 Stunden lang unter einer Stickstoffatmosphäre bei 150°C im Autoklaven gerührt. Das Reaktionsprodukt wurde einer Heißdampfextraktion mit Methanol unterworfen. Schmelzpunkt 135 bis 136°C, Ausbeute: 3,7 g.

### Beispiel 3

### Herstellung von Bisricinolsäure-1,2-N,N'-ethylendiamid

5,1 g Ricinusöl und 0,5 g 1,2-Diaminoethan wurden 5 Stunden lang unter Stickstoffatmosphäre bei 120°C gerührt. Das Produkt wurde aus Methanol umkristallisiert. Ausbeute: 2,6 g, Schmelzpunkt 83 bis 85°C.

### Beispiel 4

### Umsetzung von Bis-12-Hydroxystearinsäure-1,2-N,N'-ethylendiamid mit Hexamethylendiisocyanat

3 g des nach Beispiel 1 gewonnenen Produkts wurden unter Stickstoffatmosphäre geschmolzen und auf 155°C erhitzt. Hierzu wurden 0,79 ml Hexamethylendiisocyanat zugegeben und gut verrührt. Die Reaktionsmischung wurde 45 Minuten bei 150°C gehalten und dann auf Raumtemperatur abgekühlt. Das entstandene Polyurethanamid war thermoplastisch verarbeitbar.

### Beispiel 5

### Umsetzung von Bis-12-Hydroxystearinsäure-1,2-N,N'-ethylendiamid mit Desmodur^{(R)} E14 (einem höhermolekularen Polyisocyanat)

0.83 g Bis-12-hydroxystearinsäure-1,2-N,N'-ethylendiamid und 3,22 g Desmodur E 14 wurden unter Stickstoffatmosphäre 2 Stunden lang auf einer Temperatur von 150°C gehalten. Das entstandene Produkt war thermoplastisch verarbeitbar.

### Beispiel 6

Umsetzung von Bis-12-hydroxystearinsäure-1,6-N,N'-hexamethylendiamid mit Desmodur^{(R)} T 80 (ein Gemisch aus 80% 2,4- und 20% 2,6-Toluylendiisocyanat).

6,8 g Bis-12-hydroxystearinsäure-1,6-N,N'-hexamethylendiamid wurden geschmolzen und unter Stickstoffatmosphäre auf 160°C erhitzt. 1,74 g Desmodur T 80 (aromatisches Diisocyanat) wurden zugegeben. Die Reaktionsmischung wurde dann 4 Stunden lang auf 160°C gehalten. Das entstandene Polyurethanamid war thermoplastisch verarbeitbar.

### Beispiel 7

### Umsetzung von Bisricinolsäure-1,2-N,N'-ethylendiamid nit Hexamethylendiisocyanat

2,00 g Bisricinolsäure-1,2-N,N'-ethylendiamid wurden geschmolzen und auf 100°C unter Stickstoffatmosphäre erhitzt. Dann wurden 0,54 g Hexamethylendiisocyanat zugegeben. Die Lösung wurde 4 Stunden bei 100°C gehalten. Der erhaltene Kunststoff war bei 110°C zu einer Folie verpreßbar.

### Beispiel 8

### Umsetzung von Bis-12-hydroxystearinsäure-1,2-N,N'-ethylendiamid mit 1,4-Butandiol und Hexamethylendiisocyanat

2,45 g des in Beispiel 1 hergestellten Produkts und 0,54 g 1,4 Butandiol wurden auf 150°C erhitzt. Unter Stickstoffatmosphäre wurden 1,68 g Hexamethylendiisocyanat zugegeben. Die Reaktionsmischung wurde 1 Stunde bei 150°C gehalten. Das dabei entstandene Polyamidurethan war thermoplastisch verarbeitbar und reckbar. Aus diesem Polyamidurethan wurde bei 200°C eine Folie gepreßt. Die Folie wurde zwischen zwei Glasplatten gelegt. Dann wurden die Glasplatten auf 190°C erhitzt und leicht aufeinandergedrückt. Nach dem Abkühlen klebten die beiden Glasplatten fest zusammen.

### Beispiel 9

### Umsetzung von Bis-12-Hydroxystearinsäure-1,2-N,N'-ethylendiamid mit 1,10-Decandiol und Hexamethylendiisocyanat

1,88 g Bis-12-hydroxystearinsäure-1,2-N,N'-ethylendiamid und 0,35 g 1,10-Decandiol wurden auf 145°C erhitzt. Dann wurden unter Stickstoffatmosphäre 0,84 g Hexamethylendiisocyanat zugegeben. Die Reaktionsmischung wurde 35 Minuten bei 145°C gehalten. Das dabei entstandene Polyamidurethan ist thermoplastisch verarbeitbar und reckbar.

## Patentansprüche

1. Thermoplastische lineare Polymere, dadurch erhältlich, daß man zuerst aus ungesättigten und/oder hydroxylgruppenhaltigen, aus natürlichen Fetten und Ölen gewinnbaren Fettsäuren oder derer Estern oder Gemischen verschiedener solcher Fettsäuren und Ester durch Reaktion in stöchiometrischem Mischungsverhältnis mit bifunktionellen Verbindungen in Form von Diolen, Diaminen oder Aminoalkoholen, wobei OH-Gruppen in den Diolen durch SH-Gruppen ersetzt sein können, und gegebenenfalls durch anschließende Umsetzung vorhandener ethylenischer Doppelbindungen zu Amino-, Hydroxyl- oder Epoxidgruppen, Difettsäurediamide, Difettsäurediester, Difettsäureamidester, Monofettsäureamidamine oder Monofettsäureamidalkohole bzw. die entsprechenden Thioverbindungen als Monomerbausteine herstellt, die wenigstens zwei funktionelle Gruppen in Form von ethylenischen Doppelbindungen, Hydroxylgruppen, Epoxidgruppen oder Aminogruppen enthalten, und danach diese Monomerbausteine in äquimolaren Mengen mit bifunktionellen Verbindungen in Form von Dischwefeldichlorid, Diisocyanaten, aktivierten Dicarbonsäuren oder Dicarbonsäurederivaten, entsprechenden Thiosäuren, Phosgen oder Diketenen oder, wenn die Monomerbausteine Epoxidgruppen aufweisen, in Form von Diolen oder den entsprechenden Thioverbindungen, Dicarbonsäuren, Aminoalkoholen oder Diaminen weiter umsetzt.

2. Polymere nach Anspruch 1, dadurch erhältlich, daß als Fettsäureester Fette und Öle mit ungesättigten und/oder hydroxylgruppenhaltigen Fettsäuren im Fettsäuremuster eingesetzt werden.

3. Polymere nach Anspruch 1 oder 2, dadurch erhältlich, daß als Fettsäureester Euphorbiaöl, Olivenöl, Ricinusöl, hydriertes Ricinusöl sowie ölsäurereiches Sonnenblumenöl eingesetzt wird.

4. Polymere nach einem der Ansprüche 1 bis 3, dadurch erhältlich, daß die zur Herstellung der Monomerbausteine eingesetzte bifunktionelle Verbindung ein aliphatisches, cycloaliphatisches, aliphatisch-aromatisches oder aromatisches Diol ist.

5. Polymere nach Anspruch 4, dadurch erhältlich, daß die bifunktionelle Verbindung Glycol, 1,2-Propandiol oder 1,4-Butandiol ist.

6. Polymere nach einem der Ansprüche 1 bis 3, dadurch erhältlich, daß die zur Herstellung der Monomerbausteine eingesetzte bifunktionelle Verbindung ein aliphatisches, cycloaliphatisches, aliphatisch-aromatisches oder aromatisches Diamin ist.

7. Polymere nach Anspruch 6, dadurch erhältlich, daß die bifunktionelle Verbindung 1,2-Diaminoethan oder 1,6-Diaminohexan ist.

8. Polymere nach einem der Ansprüche 1 bis 3, dadurch erhältlich, daß die zur Herstellung der Monomerbausteine eingesetzte bifunktionelle Verbindung ein aliphatischer, cycloaliphatischer, aliphatisch-aromatischer oder aromatischer Aminoalkohol ist.

9. Polymere nach Anspruch 8, dadurch erhältlich, daß die bifunktionelle Verbindung 2-Aminoethanol oder 3-Aminopropanol ist.

10. Polymere nach einem der Ansprüche 1 bis 9, dadurch erhältlich, daß die Umsetzung mit den zur Herstellung der Monomerbestandteile eingesetzten bifunktionellen Verbindungen in einem Lösungsmittel erfolgt.

11. Polymere nach Anspruch 10, dadurch erhältlich, daß als Lösungsmittel für die Umsetzung Toluol, Xylol oder Petrolether verwendet werden.

12. Polymere nach Anspruch 10, dadurch erhältlich, daß als Lösungsmittel für die Umsetzung Methanol, Ethanol, Propanol oder Butanol verwendet werden.

13. Polymere nach einem der Ansprüche 1 bis 12, dadurch erhältlich, daß die Umsetzung mit den zur Herstellung der Monomerbausteine eingesetzten bifunktionellen Verbindungen bei Temperaturen zwischen 20 und 300°C erfolgt.

14. Polymere nach einem der Ansprüche 1 bis 13, dadurch erhältlich, daß die Umsetzung mit den zur Herstellung der Monomerbausteine eingesetzten bifunktionellen Verbindungen unter einer Inertgasatmosphäre erfolgt.

15. Polymere nach einem der Ansprüche 1 bis 14, dadurch erhältlich, daß die Umsetzung mit den zur Herstellung der Monomerbausteine eingesetzten bifunktionellen Verbindungen unter Verwendung von Katalysatoren erfolgt.

16. Polymere nach einem der Ansprüche 1 bis 15, dadurch erhältlich, daß die Umsetzung mit den zur Herstellung der Monomerbausteine eingesetzten bifunktionellen Verbindungen unter Verwendung von Antioxidantien erfolgt.

17. Polymere nach einem der Ansprüche 1 bis 16, dadurch gekennzeichnet, daß zur Umsetzung der Monomerbausteine je nach Art der freien funktionellen Gruppe Hexamethylendiisocyanat, Methylendiphenylendiisocyanat, ein Gemisch aus 80% 2,4-und 20% 2,6-Toluylendiisocyanat (Desmodur® T80), Adipinsäuredichlorid, Maleinsäuredichlorid oder 1,4-Butandiol eingesetzt werden.

18. Polymere gemäß Anspruch 1, wobei als Vertreter der zweiten Gruppe bifunktioneller Verbindungen Hexamethylendiisocyanat verwendet wird und die Monomerbausteine zusammen mit 1,4-Butandiol oder 1,10-Decandiol eingesetzt werden.

## Claims

1. Thermoplastic linear polymers that can be obtained by at first reacting in a stoichiometric ratio unsaturated and/or hydroxyl-group-containing fatty acids obtainable from natural fats and oils or their esters or mixtures of various such fatty acids and esters with bifunctional compounds in the form of diols, diamines or amino alcohols, where OH groups in the diols may have been replaced by SH groups and, if necessary, subsequently converting any possibly existing ethylenic double bonds into amino, hydroxy or epoxy groups, to give difatty acid diamides, difatty acid diesters, difatty acid amide esters, monofatty acid amide amines or monofatty acid amide alcohols or the corresponding thio compounds respectively as monomer components which contain at least two functional groups in the form of ethylenic double bonds, hydroxyl groups, epoxy groups or amino groups, and by subsequently reacting the said monomer components in equimolar amounts with bifunctional compounds in the form of disulphur dichloride, diisocyanates, activated dicarboxylic acids or dicarboxylic acid derivatives, corresponding thio acids, phosgene or diketenes or, if the monomer compounds contain epoxy groups, in the form of diols or the corresponding thio compounds, dicarboxylic acids, amino alcohols or diamines.

2. Polymers as claimed in Claim 1, which can be obtained by using as fatty acid esters fats and oils with unsaturated and/or hydroxyl-group-containing fatty acids in their fatty acid composition.

3. Polymers as claimed in Claim 1 or Claim 2, which can be obtained by using as fatty acid esters euphorbia oil, olive oil, castor oil, hydrogenated castor oil, or sunflower oil rich in oleic acid.

4. Polymers as claimed in any of Claims 1 to 3, which can be obtained by using as the bifunctional compound for preparing the monomer components an aliphatic, cycloaliphatic, alophatic-aromatic or aromatic diol.

5. Polymers as claimed in Claim 4, which can be obtained by using as the bifunctional compound glycol, 1,2-propanediol or 1,4-butanediol.

6. Polymers as claimed in any of Claims 1 to 3, which can be obtained by using as the bifunctional compound for preparing the monomer components an aliphatic, cycloaliphatic, aliphatic-aromatic or aromatic diamine.

7. Polymers as claimed in Claim 6, which can be obtained by using as the bifunctional compound 1,2-diaminoethane or 1,6-diaminohexane.

8. Polymers as claimed in any of claims 1 to 3, which can be obtained by using as the bifunctional compound for preparing the monomer components an aliphatic, cycloaliphatic, aliphatic-aromatic or aromatic amino alcohol.

9. Polymers as claimed in Claim 8, which can be obtained by using as the bifunctional compound 2-aminoethanol or 3-aminopraponol.

10. Polymers as claimed in any of Claims 1 to 9, which can be obtained by performing the reaction with the bifunctional compounds used for preparing the monomer components in a solvent.

11. Polymers as claimed in Claim 10, which can be obtained by using for the reaction toluene, xylene or petroleum ether as a solvent.

12. Polymers as claimed in Claim 10, which can be obtained by using for the reaction methanol, ethanol, propanol or butanol as a solvent.

13. Polymers as claimed in any of Claims 1 to 12, which can be obtained by carrying out the reaction with the bifunctional compounds used for preparing the monomer components at temperatures between 20 and 300°C.

14. Polymers as claimed in any of Claims 1 to 13, which can be obtained by carrying out the reaction with bifunctional compounds used for preparing the monomer components in an inert gas atmosphere.

15. Polymers as claimed in any of Claims 1 to 14, which can be obtained by using catalysts for the reaction with the bifunctional compounds used for preparing the monomer components.

16. Polymers as claimed in any of Claims 1 to 15, which can be obtained by using antioxidants for the reaction with the bifunctional compounds used for preparing the monomer components.

17. Polymers as claimed in any of Claims 1 to 16,
characterized in that
- depending on the type of free functional group in questionhexamethylene diisocyanate, methylene diphenylene diisocyanate, a mixture of 80% 2,4- and 20% 2,6-toluyene diisocyanate, (Desmodur® TBO) , adipic acid dichloride, maleic acid dichloride or 1,4-butane-diol is used for reacting the monomer components.

18. Polymers as claimed in Claim 1, wherein
hexamethylene diisocyanate is used as a member of the second group of bifunctional compounds and the monomer components are used in combination with 1,4-butanediol or 1,10-decanediol.

## Revendications

1. Polymères thermoplastiques linéaires pouvant être obtenus par le fait que l'on prépare dans un premier temps, à partir d'acides gras insaturés et/ou contenant des groupes hydroxyles, pouvant être obtenus à partir d'huiles et de graisses naturelles, ou de leurs esters ou de mélanges de différents acides gras et esters de ce type, par réaction, selon une proportion stoechiométrique, avec des composés bifonctionnels sous la forme de diols, de diamines ou d'amino-alcools, les groupes OH dans les diols pouvant être remplacés par des groupes SH, et éventuellement ensuite par transformation des doubles liaisons éthyléniques existantes en groupes amino, hydroxyles ou époxydes, des diamides de diacide gras, des diesters de diacide gras, des esters-amide de diacide gras, des amines d'amides de mono-acide gras ou des alcools d'amides de mono-acide gras ou les composés thio correspondants, en tant qu'éléments monomères constitutifs, qui contiennent aux moins deux groupements fonctionnels sous forme de doubles liaisons éthyléniques, de groupes hydroxyles, de groupes époxydes ou de groupes amino, et que l'on transforme ensuite ces éléments monomères constitutifs en quantités équimolaires avec des composés bifonctionnels sous forme de chlorure de soufre, de diisocyanates, d'acides dicarboxyliques activés ou de dérivés d'acide dicarboxylique, de thio-acides correspondants, de phosgène ou de dicétènes ou, lorsque les éléments monomères constitutifs comportent des groupes époxydes, sous la forme de diols ou des composés thio correspondants, d'acides dicarboxyliques, d'amino- alcools ou de diamines.

2. Polymères suivant la revendication 1, pouvant être obtenus par le fait que l'on utilise comme esters d'acide gras des graisses et des huiles avec des acides gras insaturés et/ou contenant des groupes hydroxyles dans l'échantillon d'acide gras.

3. Polymères suivant la revendication 1 ou 2, pouvant être obtenus par le fait que l'on utilise comme esters d'acide gras, de l'huile d'euphorbe, de l'huile d'olive, de l'huile de ricin, de l'huile de ricin hydrogénée ainsi que de l'huile de tournesol riche en acide oléique.

4. Polymères suivant l'une des revendications 1 à 3, pouvant être obtenus par le fait que le composé bifonctionnel utilisé pour préparer les éléments monomères consistutifs est un diol aliphatique, cycle-aliphatique, aliphatique-aromatique ou aromatique.

5. Polymères suivant la revendication 4, pouvant être obtenus par le fait que le composé bifonctionnel est du glycol, du 1,2-propanediol ou du 1,4- butanediol.

6. Polymères suivant l'une des revendications 1 à 3, pouvant être par le fait que le composé bifonctionnel utilisé pour préparer les éléments monomères constitutifs est une diamine aliphatique, cyclo-aliphatique, aliphatique-aromatique ou aromatique.

7. Polymères suivant la revendication 6, pouvant être obtenus par le fait que le composé bifonctionnel est du 1,2-diaminoéthane ou du 1,6-diaminohéxane.

8. Polymères suivant l'une des revendications 1 à 3, pouvant être obtenus par le fait que le composé bifonctionnel utilisé pour préparer les éléments monomères constitutifs est un amino-alcool aliphatique, cyclo-aliphatique, aliphatique-aromatique ou aromatique.

9. Polymères suivant la revendication 8, pouvant être obtenus par le fait que le composé bifonctionnel est du 2-aminoéthanol ou du 3-aminopropanol.

10. Polymères suivant l'une des revendications 1 à 9, pouvant être obtenus par le fait que la transformation avec les composés bifonctionnels utilisés pour préparer les éléments monomères constitutifs s'effectue dans un solvant.

11. Polymères suivant la revendication 10, pouvant être obtenus par le fait que le solvant utilisé pour la transformation est du toluène, du xylène ou de l'éther de pétrole.

12. Polymères suivant la revendication 10, pouvant être obtenus par le fait que le solvant utilisé pour la transformation est du méthanol, de l'éthanol, du propanol ou du butanol.

13. Polymères suivant l'une des revendications 1 à 12, pouvant être obtenus par le fait que la transformation avec les composés bifonctionnels utilisés pour préparer les éléments monomères constitutifs s'effectue à des températures entre 20 et 300°C.

14. Polymères suivant l'une des revendications 1 à 13, pouvant être obtenus par le fait que la transformation avec les composés bifonctionnels utilisés pour préparer les éléments monomères constitutifs s'effectue sous une atmosphère inerte.

15. Polymères suivant l'une des revendications 1 à 14, pouvant être obtenus par le fait que la transformation avec les composés bifonctionnels utilisés pour préparer les éléments monomères constitutifs s'effectue en utilisant des catalyseurs.

16. Polymères suivant l'une des revendications 1 à 15, pouvant être obtenus par le fait que la transformation avec les composés bifonctionnels utilisés pour préparer les éléments monomères constitutifs s'effectue en utilisant des antioxygènes.

17. Polymères suivant l'une des revendications 1 à 16, pouvant être obtenus par le fait que pour la transformation des éléments monomères constitutifs, on utilise, selon le type du groupement fonctionnel libre, de l'hexaméthylène-diisocyanate, du méthylène-diphénylène- diisocyanate, un mélange de 80 % de 2,4-toluylène-diisocyanate et de 20 % de 2,6-toluylène-diisocyanate (Desdomur ® T80), du dichlorure d'adipoyle, du dichlorure de maléoyle ou du 1-4-butanediol.

18. Polymères suivant la revendication 1, de l'hexaméthylène-diisocyanate étant utilisé comme représentant du deuxième groupe de composés bifonctionnels et les éléments monomères constitutifs étant utilisés avec du 1,4-butanediol ou du 1,10-décanediol.
